# FASCICULE DE BREVET EUROPEEN

(11) **EP 1 496 869 B1**
(45) Date de publication et mention de la délivrance du brevet: **18.02.2009**
(21) Numéro de dépôt: 03740656.8
(22) Date de dépôt: 23.04.2003
(51) Int. Cl.: A61K 9/26

(54) **PARTICULES ENROBEES A LIBERATION PROLONGEE ET COMPRIMES LES CONTENANT**
BESCHICHTETE TEILCHEN MIT VERZÖGERTER FREISETZUNG UND DIESE ENTHALTENDE TABLETTEN
COATED PARTICLES WITH PROLONGED RELEASE AND TABLETS CONTAINING SAME

(30) Priorité: 23.04.2002 FR 0205077
(43) Date de publication de la demande: 19.01.2005
(73) Titulaire: ETHYPHARM, 78550 Houdan (FR)
(72) Inventeur: CHENEVIER, Phillippe, Montreal, Québec H2V 4K8 (CA); MARECHAL, Dominique, Laval, Québec H7H 2W8 (CA)
(74) Mandataire: Koch, Gustave
(86) Numéro de dépôt international: PCT/FR2003/001284
(87) Numéro de publication internationale: WO 2003/090724

(56) Documents cités:
- WO-A-96/39127
- WO-A-99/44580
- FR-A- 2 385 388
- BODMEIER R: "TABLETING OF COATED PELLETS" EUROPEAN JOURNAL OF PHARMACEUTICS AND BIOPHARMACEUTICS, ELSEVIER SCIENCE PUBLISHERS B.V., AMSTERDAM, NL, vol. 43, no. 1, 1997, pages 1-8, XP000678667 ISSN: 0939-6411 cité dans la demande

## Description

L'invention est relative à des particules enrobées et, plus précisément à des granules ou granulés enrobés à libération prolongée, à leur procédé de préparation et à des comprimés multiparticulaires comprenant lesdites particules enrobées.

Les formes multiparticulaires à libération prolongée sont bien connues dans l'art antérieur.

Ces formes existent en particulier sous des formes unitaires telles que des gélules ou des comprimés multiparticulaires.

Par comprimé multiparticulaire, on entend un comprimé comprenant, d'une part des particules contenant au moins un principe actif, enrobées individuellement par un film polymérique modulant la libération du principe actif sur une période pouvant aller de 8 à 24 heures, et d'autre part des excipients de compression.

Les particules enrobées sont d'abord mélangées avec les excipients de compression, le mélange étant ensuite comprimé de manière à conduire à une forme unitaire homogène.

Lors de l'étape de compression, l'enrobage polymérique subit des contraintes importantes, qui sont telles que des fissures ou ruptures peuvent se produire, provoquant une fuite et la mise à disposition immédiate de la totalité du principe actif.

Le film polymérique doit donc être suffisamment souple et déformable pour supporter la compression.

Pour évaluer les propriétés et caractéristiques mécaniques du film polymérique, on peut utiliser des paramètres tels que la résistance à la rupture ou le pourcentage d'élongation. Ces caractéristiques mécaniques peuvent être déterminées par les méthodes décrites dans les normes DIN53455 et ISO/RI 184.

Les polymères acryliques sont une famille de polymères présentant des propriétés de déformation qui permettent la réalisation de comprimés multiparticulaires.

Dans la demande EP 1032374, la Demanderesse a décrit des sphéroïdes comprenant dans le noyau ou en couche, un excipient thermoplastique dont la consistance est pâteuse à semi-solide à 20°C, et un film souple et déformable dont le pourcentage d'élongation est supérieur ou égal à 50%, et qui est constitué par un copolymère neutre d'esters d'acides acryliques, par exemple celui connu sous la marque Eudragit® NE30D dont le pourcentage d'élongation est de 600% et dont la résistance à la compression est de 8N/m².

Les polymères présentant des paramètres d'élongation ou de résistance à la compression médiocres, n'absorbent normalement pas les contraintes mécaniques liées à la compression (International Journal of Pharmaceutics, 143, 13-23 (1996)).

De nombreuses publications décrivent les polymères cellulosiques comme étant cassants et peu élastiques. C'est par exemple le cas de l'éthycellulose dont la capacité d'élongation est habituellement inférieure à 15% , ce qui rend difficile son utilisation comme agent enrobant pour des particules destinées à être comprimées (Eur. J. Pharm. Biopharm., 43, 1-8 (1997)).

Des études de films libres à base d'éthylcellulose montrent que l'addition d'agents plastifiants ou d'agents solubles dans le film d'enrobage, et la mise en oeuvre d'une étape de maturation sous certaines conditions de température et d'humidité (Int. J. Pharm. 104, 203-213, (1994), Acta Pharm. Technol. 35(4), 232-237, (1989)) permettent éventuellement d'améliorer les propriétés des films à base de dérivés cellulosiques. FR 2 385 388 décrit l'utilisation d'une substance cireuse, comme par exemple polyoxyéthylèneglycol, comme excipient du comprimé pour la protection des particules fragiles.

Cette amélioration n'est toutefois pas suffisante. Par ailleurs, le profil de libération du principe actif est directement influencé par la composition du film d'enrobage.

Il serait particulièrement avantageux de disposer de moyens permettant de les rendre systématiquement utilisables pour la constitution de films d'enrobage, sans modifier leur composition, ni influencer leur profil de libération du principe actif.

Or, la Demanderesse a eu le mérite de trouver que ce but pouvait être atteint et qu'il devenait possible d'obtenir des particules enrobées à libération prolongée et à enrobage constitué d'au moins un polymère cellulosique telles, que le profil de libération d'un comprimé multiparticulaire à base de ces particules soit identique à celui des particules enrobées avant compression, les deux profils de libération étant déterminés dans des conditions de mise en oeuvre (milieu de dissolution, appareil, méthode) similaires, dès lors que l'on fait comporter auxdites particules un enrobage protecteur à base d'au moins un agent thermoplastique de point de fusion de 25 à 100°C appliqué sur l'enrobage à base de polymère cellulosique.

On considère que deux profils de libération sont similaires dès lors que la variation entre les moyennes des valeurs, mesurées pour chacun des profils à chaque temps de prélèvement est inférieure ou égale à plus ou moins 15%, de préférence plus ou moins 10%, c'est-à-dire dans le cas présent, entre la valeur moyenne obtenue pour les particules enrobées avant compression et celle obtenue pour le comprimé multiparticulaire à base desdites particules.

Si la variation en question est supérieure à 15%, on considère que le profil de libération est significativement modifié.

Il s'ensuit que les particules enrobées à libération prolongée, conformes à l'invention, qui comprennent
- un noyau comprenant un principe actif et au moins un agent liant et
- un film d'enrobage à base d'au moins un polymère cellulosique, seul ou en mélange avec un plastifiant,
sont caractérisées par le fait qu'elles comportent un enrobage protecteur à base d'au moins un agent thermoplastique dont la température de fusion est d'environ 25°C à environ 100°C et qui est appliqué sur le film d'enrobage à base d'au moins un polymère cellulosique.

Les particules conforme à l'invention sont constituées par des granules ou des granulés, selon le procédé d'obtention du noyau actif.

Les granulés sont obtenus par granulation sèche ou humide et les granules par montage du principe actif sur un neutre.

Le noyau de la particule comprend au moins un principe actif choisi parmi ceux du groupe comprenant les sédatifs gastrointestinaux, les antiacides, les antalgiques, les antiinflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les antidiarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste.

Ce principe actif, initialement sous forme de poudre ou de microcristaux est utilisé à l'état sec pour la préparation de granulés, et sous forme de solution ou suspension dans un solvant aqueux ou organique pour le montage sur neutres.

Avantageusement, les noyaux comprennent un agent liant qui permet de lier la poudre ou les microcristaux de principe actif et les autres constituants éventuels, afin de donner des particules de taille suffisante pour faciliter l'opération d'enrobage.

L'agent liant peut être choisi dans le groupe comprenant notamment les polymères cellulosiques, les polymères acryliques, les povidones, les copovidones, les polyvinylalcools , l'acide alginique, l'alginate de sodium, l'amidon, l'amidon prégélatinisé, les sucroses et leurs dérivés, la gomme guar, les polyéthylèneglycols et leurs mélanges.

Parmi les polymères cellulosiques, on choisira avantageusement l'éthycellulose, l'hydroxypropylcellulose et l'hydroxypropylméthylcellulose, seuls ou en mélange.

Parmi les polymères acryliques, on choisit avantageusement le copolymère ammonio-méthacrylate, les polymères et copolymères d'acide acrylique et méthacryliques, les polyacrylates et les polyméthacrylate, utilisés seuls ou en mélange.

L'agent liant est présent dans des proportions pouvant aller jusqu'à 15 % en poids, de préférence jusqu'à 10 % en poids par rapport au poids des particules non enrobées.

Le noyau comprend de façon optionnelle, un diluant et un agent antistatique.

Le diluant peut être choisi dans le groupe comprenant notamment les dérivés cellulosiques et préférentiellement la cellulose microcristalline, les amidons seuls, le lactose, les polyols et préférentiellement le mannitol.

Le noyau peut également être un neutre constitué d'un mélange d'amidon et de saccharose, ou bien de cellulose microcristalline.

Le diluant est présent dans des proportions pouvant aller jusqu'à 95% en poids, de préférence jusqu'à 50% en poids par rapport au poids des particules non enrobées.

Son rôle est d'augmenter la masse totale de particules à enrober et d'obtenir une population de particules de taille homogène.

L'agent antistatique peut être choisi dans le groupe comprenant la silice colloïdale, notamment celle commercialisée sous la marque Aerosil^{®}, et préférentiellement la silice précipitée, notamment celle commercialisée sous le nom Syloïd^{®} FP244, le talc micronisé ou non et leurs mélanges.

L'agent antistatique est présent dans des proportions pouvant aller jusqu'à 10% en poids, de préférence jusqu'à 3% en poids par rapport au poids des particules non enrobées.

Il améliore la fluidisation de la matière lors de l'utilisation d'un lit d'air fluidisé, en particulier dans le cas d'une granulation de poudre.

Les noyaux comprenant le principe actif sont ensuite enrobés à l'aide d'une première composition enrobante.

Le film d'enrobage ainsi obtenu permet la libération prolongée du principe actif.

Il est constitué d'au moins un polymère cellulosique, seul ou en mélange avec un plastifiant.

Parmi les polymères cellulosiques, on choisit de préférence l'éthylcellulose.

Ce film d'enrobage est appliqué par pulvérisation d'une solution, d'une suspension ou d'une dispersion colloïdale de ce polymère dans un solvant, pour former un film continu recouvrant la totalité de la surface de chaque particule, et ce, quel que soit l'état de surface de cette dernière, en quantité suffisante pour permettre la libération prolongée du principe actif sur une durée pouvant aller de 8 à 24 heures.

Le polymère d'enrobage est présent dans des proportions pouvant aller jusqu'à 50%, de préférence jusqu'à 20%, calculées en gain de poids par rapport à la masse de noyaux à enrober.

Le solvant choisi pour pulvériser le polymère cellulosique peut être l'eau, un solvant organique, tel que l'éthanol, l'isopropanol, l'acétone, ou un mélange de solvants.

Le polymère se trouve alors sous forme de solution, suspension, ou dispersion, dans le solvant ou mélange de solvants. Il est de préférence sous forme de solution dans un solvant organique, de préférence dans l'isopropanol.

La composition enrobante comprend également de façon optionnelle, un agent formant des pores, un agent plastifiant, un agent tensioactif, un agent antistatique, un lubrifiant.

L'agent formant des pores permet de moduler la libération du principe actif, et en particulier de l'accélérer.

Cet agent doit être soluble dans les milieux de pH inférieur ou égal à 5. Il peut être par exemple choisi parmi les sucres, les polyols, les polymères ou copolymères des acides acrylique ou méthacrylique, les dérivés cellulosiques hydroxylés, les povidones et les polyvinylalcools.

L'agent plastifiant est choisi dans le groupe comprenant le triethyl citrate, l'acétyltributyl citrate, la triacétine, le tributyl citrate, le diethylphtalate, les polyethylènes glycols, les polysorbates, les glycérides mono-et diacétylés et leurs mélanges.

Sa fonction est d'abaisser la température de transition vitreuse du film d'enrobage et d'en améliorer les propriétés mécaniques.

Il est utilisé dans une proportion d'au plus 40%, de préférence entre 15 à 30%, exprimé en poids par rapport au poids sec de polymère.

L'agent tensioactif est choisi parmi les tensioactifs anioniques, cationiques, non ioniques ou amphotères.

L'agent antistatique, éventuellement utilisé, est mis en oeuvre pour éviter les problèmes liés à l'électricité statique. Il est choisi dans le groupe comprenant le talc micronisé ou non micronisé, la silice colloïdale (Aerosil^{®}200), la silice traitée (Aerosil^{®} R972), la silice précipitée (Syloïd^{®} FP244) et leurs mélanges.

Il est mis en oeuvre en une proportion d'au plus 10% en poids, de préférence comprise entre 0 et 3%, et plus préférentiellement encore inférieure à 1% en poids.

Le lubrifiant est choisi dans le groupe comprenant le stéarate de magnésium, l'acide stéarique, le sodium stearyl fumarate, les polyoxyéthylèneglycols micronisés (Macrogol 6000 micronisé), le benzoate de sodium et leurs mélanges.

Une couche polymérique optionnelle peut être appliquée entre le noyau et le film polymérique fonctionnel pour isoler le noyau, comportant le principe actif, de la couche de polymère cellulosique qui permet la libération prolongée du principe actif.

Le polymère constitutif de la couche optionnelle peut être choisi parmi les mêmes polymères que ceux utilisés comme liant; en particulier, il peut être identique ou différent de celui utilisé comme liant dans le noyau comportant le principe actif.

L'avantage apporté par cette couche optionnelle est de permettre d'isoler le principe actif du polymère fonctionnel qui en permet la libération prolongée,
- lorsqu'il y a incompatibilité entre le principe actif et le polymère fonctionnel,
- lorsque le principe actif est instable au pH de la solution ou suspension utilisée pour appliquer le polymère fonctionnel,
- lorsque le principe actif présente une solubilité importante dans le solvant utilisé pour l'application du polymère fonctionnel, ce qui peut être à l'origine d'une "migration" du principe actif dans le film polymère rendant ainsi difficile l'obtention d'une libération prolongée du principe actif.

La quantité de polymère appliquée est comprise entre 1 et 10% de préférence entre 2 et 5% en gain de poids par rapport à la masse de noyaux comportant le principe actif mise en oeuvre.

Conformément à l'invention, la couche de polymère cellulosique permettant la libération prolongée du principe actif est recouverte par un enrobage la protégeant.

Cet enrobage protecteur est à base d'au moins un agent thermoplastique dont la température de fusion est d'environ 25°C à environ 100°C et éventuellement d'un lubrifiant.

L'agent thermoplastique est choisi dans le groupe comprenant les huiles partiellement hydrogénées, la cire d'abeille, la cire de carnauba, les cires de paraffine, les cires de silicone, les alcools gras, les acides gras en C12-C18, les glycérides semi-synthétiques solides, les monoester, diesters ou triesters du glycérol, les polyoxyéthylèneglycols, les glycérides polyoxyéthylénés glycosylés et leurs mélanges.

L'enrobage protecteur a pour fonction d'absorber les contraintes dues à la compression, et d'éviter une déformation, une altération ou une rupture du film d'enrobage constitué par le polymère cellulosique et dont la fonction est d'assurer la libération prolongée du principe actif.

L'agent thermoplastique est utilisé dans un proportion pouvant aller jusqu'à 100% en gain de poids par rapport à la masse de granulé à enrober, de préférence entre 10 et 50% en gain de poids.

Il est, de préférence choisi parmi les excipients hydrophiles de balance hydrophile/lipophile (HLB) supérieure à 10, de façon à ne pas modifier le profil de libération du principe actif.

L'enrobage protecteur peut, en outre, comprendre un agent anti-statique.

La répartition granulométrique des particules conformes à l'invention permet leur utilisation dans la fabrication de comprimés multiparticulaires.

Avantageusement, la taille des particules est inférieure à 700µm, étant entendu que la taille d'au moins 50%, de préférence d'au moins 70% des particules est comprise entre 150 et 500µm, et que la taille de moins de 15% des particules est inférieure à 150µm.

La taille des particules est déterminée par les méthodes conventionnelles, par exemple à l'aide d'un jeu de tamis de maille calibré, ou par diffraction d'un laser.

Les particules enrobées à libération prolongée ainsi préparées, de par leurs propriétés mécaniques, sont particulièrement aptes à entrer dans la constitution de comprimés multiparticulaires.

Le comprimé multiparticulaire conforme à l'invention est caractérisé par le fait qu'il est à base des particules enrobées conformes à l'invention, ce comprimé comprenant en outre un mélange d'excipients comprenant
- un agent de désintégration et/ou un agent gonflant,
- au moins un agent diluant,
- un lubrifiant et
- éventuellement, un agent antistatique, un agent perméabilisant, des édulcorants, des arômes et des colorants.

L'agent de désintégration est choisi dans le groupe comprenant la carboxyméthylcellulose sodique réticulée désignée dans le métier par le terme croscarmellose, la crospovidone et leurs mélanges.

L'agent gonflant est choisi dans le groupe comprenant la cellulose microcristalline, les amidons et les amidons modifiés.

L'agent diluant peut être choisi dans le groupe comprenant les dérivés cellulosiques et préférentiellement la cellulose microcristalline, le lactose, les polyols et préférentiellement le mannitol.

Le lubrifiant est choisi dans le groupe comprenant le stéarate de magnésium, l'acide stéarique, le sodium stearyl fumarate, les polyoxyéthylèneglycols (Macrogol 6000 micronisé), le benzoate de sodium et leurs mélanges.

Le lubrifiant, en totalité ou en partie, est dispersé au sein du mélange d'excipients de compression et/ou pulvérisé à la surface du comprimé au moment de la compression.

L'agent antistatique peut être choisi dans le groupe comprenant la silice colloïdale, notamment celle commercialisée sous la marque Aerosil^{®}, et préférentiellement la silice précipitée, notamment celle commercialisée sous le nom Syloïd^{®} FP244, le talc micronisé ou non, et leurs mélanges.

L'agent perméabilisant est choisi dans le groupe comprenant notamment les silices ayant une grande affinité pour les solvants aqueux, telles que la silice précipitée plus connue sous le nom de marque Syloïd^{®}, les maltodextrines, les β-cyclodextrines et leurs mélanges.

Il permet la création d'un réseau hydrophile qui facilite la pénétration de la salive et contribue ainsi à une meilleure désagrégation du comprimé.

L'édulcorant peut être choisi dans le groupe comprenant notamment l'aspartame, l'acésulfame de potassium, le saccharinate de sodium, la néohespéridine dihydrochalcone, le sucralose, le monoammonium glycyrrhizinate et leurs mélanges.

Les arômes et colorants sont ceux utilisés habituellement en pharmacie pour la préparation de comprimés.

Dans les comprimés multiparticulaires conformes à l'invention, la proportion du mélange d'excipients par rapport aux particules enrobées est de 0,4 à 10, de préférence comprise entre 1 et 5 parties en poids, ledit mélange d'excipients comprenant :
- de 1 à 15% , de préférence de 2 à 7% en poids d'agent de désintégration et/ou d'agent gonflant
- de 30 à 90%, de préférence de 40 à 70% en poids par rapport à la masse du comprimé, d'agent diluant,
- de 0,02 à 2%, de préférence de 0,5 à 1% en poids de lubrifiant, par rapport à la masse du comprimé,
- de 0,5% à 5% en poids d'agent perméabilisant par rapport à la masse du comprimé.

Dans un mode de réalisation préféré, le comprimé multiparticulaire selon l'invention est un comprimé orodispersible qui se désintègre dans la bouche, au contact de la salive, en moins de 60 secondes, de préférence en moins de 40 secondes, en formant une suspension aisée à avaler.

Le temps de désintégration correspond à la durée comprise entre le moment de la mise en place du comprimé dans la bouche au contact de la salive et le moment de la déglutition de la suspension résultant de la désintégration sans mastication du comprimé au contact de la salive.

L'agent diluant est choisi parmi les agents solubles à propriétés liantes, constitués par des polyols de moins de 13 atomes de carbone et se présentant soit sous forme d'un produit directement compressible dont le diamètre moyen des particules est de 100 à 500 µm, soit sous la forme d'une poudre dont le diamètre moyen des particules est inférieur à 100 µm, ces polyols étant de préférence choisis dans le groupe comprenant le mannitol, le xylitol, le sorbitol et le maltitol, utilisés sous la forme du produit directement compressible, alors que, dans le cas où il y aurait au moins deux agents solubles diluants à propriétés liantes, l'un serait présent sous la forme directement, compressible et l'autre sous la forme d'une poudre, le polyol pouvant être le même, les proportions de polyol directement compressible et de polyol poudre étant de 99/1 à 20/80, de préférence de 80/20 à 20/80.

L'invention a également pour objet un procédé de préparation des particules enrobées à libération prolongée.

Le procédé en question comprend, conformément à l'invention, les étapes suivantes :
- préparation par granulation humide ou montage sur neutres, d'un noyau comprenant le principe actif,
- enrobage des noyaux ainsi obtenus par pulvérisation d'une composition d'enrobage constituée d'au moins un polymère cellulosique,
- enrobage des particules enrobées ainsi obtenues par pulvérisation d'une composition d'enrobage protecteur à base d'un excipient de type thermoplastique,
- séchage.

Selon ce mode de mise en oeuvre, les étapes successives du procédé peuvent être réalisées dans des appareils différents ou dans le même appareil.

Dans un premier mode de réalisation avantageux, les noyaux comprenant le principe actif sont préparés par granulation selon les étapes suivantes :
- mélange à sec du principe actif sous forme de poudre ou de microcristaux, éventuellement avec l'agent diluant et un agent antistatique,
- granulation du mélange obtenu par pulvérisation d'une solution de l'agent liant,
- séchage.

Pour la granulation, un granulateur haute énergie, un mélangeur planétaire ou un lit d'air fluidisé sont avantageusement utilisés.

Dans le cas de la granulation en lit d'air fluidisé, le mélange de poudre contenant le principe actif et, éventuellement, le diluant et l'agent antistatique, est introduit dans l'appareil puis granulé par pulvérisation sur ledit mélange de poudre d'une solution ou suspension d'excipients comprenant au moins un agent liant.

Selon un autre mode de réalisation avantageux, le polymère mis en oeuvre lors de l'étape de granulation et celui mis en oeuvre lors de l'étape d'enrobage sont identiques. Dans ce cas, l'étape de granulation diffère de l'étape d'enrobage par différents paramètres, tels que le débit de pulvérisation du mélange d'excipients et la pression d'atomisation dudit mélange.

Ainsi, lors de l'étape de granulation, le débit de pulvérisation de la suspension d'excipients est plus élevé que lors de l'étape d'enrobage, alors que la pression d'atomisation de la suspension d'excipients est moins élevée lors de l'étape de granulation que lors de l'étape d'enrobage.

En pratique, à l'échelle du laboratoire au sein d'un appareil à lit d'air fluidisé par exemple du type GLATT GPCG3, le débit de pulvérisation du mélange d'excipients est, lors de l'étape de granulation, de 15 à 30 grammes/minute, et la pression d'atomisation de 1 à 2,5 bars.

Lors de l'étape d'enrobage, le débit de pulvérisation de la suspension d'enrobage est de 10 à 25 grammes/minute, tandis que la pression d'atomisation est de 1,5 à 3 bars.

Avantageusement, une proportion de 10 à 30 % du mélange d'excipients est pulvérisée pendant l'étape de granulation, le complément à 100 % étant pulvérisé pendant l'étape d'enrobage.

Dans un second mode de réalisation des noyaux comprenant le principe actif, ceux-ci sont préparés par montage sur neutres selon les étapes suivantes :
- pulvérisation sur des neutres d'une solution ou une suspension du principe actif, contenant le liant solubilisé,
- séchage.

Les noyaux ainsi obtenus sont ensuite enrobés par pulvérisation successive des différentes compositions d'enrobage, puis séchés.

L'enrobage polymérique constitué d'un dérivé cellulosique est pulvérisé sous forme de solution, de suspension ou de dispersion colloidale.

L'excipient thermoplastique peut être appliqué par pulvérisation sous forme d'une solution dans un solvant aqueux ou organique.

Dans un mode de réalisation particulier, l'excipient thermoplastique peut être chauffé à une température supérieure à son point de fusion, puis pulvérisé sous forme liquide sans solvant.

Dans ce cas, on installe un dispositif de pulvérisation thermostaté permettant d'éviter l'obstruction des tuyaux.

L'ensemble des étapes du procédé conforme à l'invention peuvent être réalisées dans une turbine à dragéification, dans une turbine perforée ou en lit d'air fluidisé.

Dans un mode de réalisation préféré du procédé conforme à l'invention, toutes les étapes de préparation du noyau actif et d'enrobage sont effectuées en lit d'air fluidisé.

Le lit d'air fluidisé est équipé d'une buse de pulvérisation dont la position et l'orientation de pulvérisation peuvent être choisies. La pulvérisation est désignée par les termes de métier « top spray », « bottom spray » ou « tangential spray ».

Ce choix permet de maîtriser la cinétique de croissance des particules et d'éviter des phénomènes de collage, liés à la nature du principe actif, à la composition de la composition liante ou enrobante pulvérisée, et des divers paramètres du procédé (température, pression d'air par exemple, débit de solution).

L'invention porte également sur un procédé de préparation des comprimés multiparticulaires comprenant les particules enrobées à libération prolongée.

Ce procédé comporte, conformément à l'invention, les étapes suivantes :
- mélange à sec des particules enrobées, obtenues selon le procédé décrit précédemment, avec les excipients de compression,
- compression du mélange ainsi obtenu, pour constituer une forme unitaire.

La compression du mélange peut être réalisée sur une machine à comprimer alternative ou rotative.

Les contraintes exercées lors de l'étape de compression peuvent varier de 5kN à 50kN, de préférence de 5 kN à 15 kN.

La dureté des comprimés ainsi obtenus est de préférence de 1 à 10kp, plus préférentiellement de 1 à 5kp, mesurée selon la méthode de la Pharmacopée Européenne (2.9.8), 1kp étant égal à 9,8N.

De préférence, on adapte la dureté du comprimé multiparticulaire, d'une part de façon à obtenir une friabilité inférieure à 2%, mesurée selon la méthode de la Pharmacopée Européenne, tout en conservant un profil de dissolution identique à celui des particules enrobées et, d'autre part de façon à obtenir un temps de désintégration dans la bouche inférieur ou égal à 60 secondes, de préférence inférieur ou égal à 40 secondes.

Les comprimés peuvent avoir un diamètre de 6mm à 17 mm. Leur forme peut être ronde, ovale, oblongue, présentant une surface plate ou concave, et, éventuellement, des gravures.

Dans le cas de comprimés orodispersibles, on pourra utiliser des poinçons « polo », c'est-à-dire des poinçons permettant d'obtenir des comprimés ronds, plats et présentant une concavité au centre des deux faces.

Les comprimés ont une masse qui est, de préférence, comprise entre 0,1 et 2,0 grammes.

L'invention sera mieux comprise à l'aide des exemples de réalisation des particules enrobées et des comprimés multiparticulaires conformes à l'invention. Ces exemples sont donnés uniquement à titre d'illustration et de modes de réalisation avantageux de l'invention et n'en constituent nullement une limitation.

### Exemple 1 :

Granulés enrobés comprenant de l'oxycodone HCI comme principe actif.

### Granulation

500 grammes d'oxycodone HCl sont mélangés avec 15 grammes de Syloid® 244 FP [soit 3%(w/w) par rapport à la masse d'oxycodone HCI].

Dans un mélangeur planétaire, on granule le mélange de poudres à l'aide d'une solution de mouillage constituée d'ethylcellulose N7, à 8%(w/w) dans l'isopropanol.

Le grain formé est séché en étuve à 45°C pendant 5 heures, puis tamisé sur un tamis de diamètre de 0,9 mm.

### Enrobage

On réalise l'opération d'enrobage dans un lit d'air fluidisé GLATT GPCG-3 équipé d'un insert Würster (« bottom spray »).

La masse obtenue est enrobée par pulvérisation de la même solution qu'à l'étape précédente.

On applique une quantité de polymère correspondant à 42%(w/w), calculée en gain de poids par rapport à la masse de granulés non enrobés de départ.

La répartition granulométrique des particules enrobées E1 est dans le Tableau 1.

**Tableau 1**

| Ouverture de maille | E1 |
|---|---|
| >0.710mm | 20.7% |
| 0.600mm-0.710mm | 11.5% |
| 0.500mm-0.600mm | 12.5% |
| 0.355mm-0.500mm | 17.4% |
| 0.180mm-0.355mm | 25.8% |
| 0.075mm-0.180mm | 7.9% |
| <0.075mm | 4.2% |

Le rendement en masse est de 96.32%(w/w).

La composition des granulés enrobés E1 figure dans le Tableau 2 :

**Tableau 2**

| | %(w/w) |
|---|---|
| Oxycodone HCl | 65.46 |
| Syloid® 244FP | 1.96 |
| Ethylcellulose N7 | 32.60 |
| Alcool isopropylique | -- |

### Exemple 2 :

Comprimés multiparticulaires orodispersibles comprenant de l'oxycodone HCl comme principe actif.

### Compression

On utilise une machine à comprimer alternative "Manesty F3 press" équipée de poinçons POLO de 8 mm de diamètre.

Les granulés enrobés obtenus selon l'exemple 1 sont mélangés à des excipients de compression.

On réalise deux types de comprimés, respectivement C1 et C2 comprenant des proportions relatives différentes de granulés enrobés d'oxycodone HCl.

Les comprimés C1 et C2 sont dosés respectivement à 19,8 et à 10 mg d'oxycodone HCI, selon les indications du Tableau 3 :

**Tableau 3**

| | **C1** | **C2** |
|---|---|---|
| **Constituants** | **% en poids** | **% en poids** |
| Granulés enrobés d'oxycodone HCl | 19,8 | 10.0 |
| Mannitol 300 | 33.2 | 37.3 |
| Mannitol 60 | 33.2 | 37.3 |
| Crospovidone | 9.8 | 10.0 |
| Arôme orange | 1.0 | 1.0 |
| Syloid® 244FP | 1.0 | 0.4 |
| Stéarate de magnésium | 2.0 | 2.0 |
| Aspartame | -- | 2 |
| **Total** | **100** | **100** |

Les comprimés C1 et C2 présentent les caractéristiques indiquées au Tableau 4 :

**Tableau 4**

| | **C1** | **C2** |
|---|---|---|
| Proportion théorique granulés/comprimé | 20% | 10% |
| Poids (mg) | 156.0 | 308.6 |
| Dureté (kP) | 1,5 | 1,5 |
| Friabilité | non déterminé | non déterminé |
| Temps de désintégration en bouche | 10-15 sec. | 15 sec. |

On établit un profil de dissolution comparatif entre les granulés enrobés de l'exemple 1 (désignés par E1) et les comprimés de formule C1 et C2 dans les conditions suivantes :
- appareil : USP type II
- vitesse des pales : 100 tours par minute
- volume : 900 ml
- température : 37.0°C ±0.5°C
- détection: Spectrophotométrie UV directe à 244 nm
- milieu de dissolution: HCl 0.01 N (pH = 2)

Les résultats enregistrés sont réunis dans le Tableau 5 :

**Tableau 5**

| Oxycodone libérée (%(w/w) | | | |
|---|---|---|---|
| Temps (heure) | **E1** | **C1** | **C2** |
| 0.5 | 16 | 37 | 43 |
| 1 | 30 | 53 | 58 |
| 2 | 47 | 74 | 78 |
| 4 | 69 | 92 | 95 |
| 6 | 78 | 95 | 98 |
| 8 | 81 | 96 | 99 |
| 10 | 81 | 96 | 99 |

### Conclusion

Il est possible de déduire des résultats réunis au Tableau 5, que la compression provoque une rupture du film d'éthylcellulose, ce qui entraîne une accélération de la libération du principe actif.

Il s'ensuit que les propriétés mécaniques du film d'enrobage ne sont pas adaptées à la compression sous forme de comprimé orodispersible à libération prolongée.

### Exemple 3 :

Granules enrobés comprenant de la théophylline comme principe actif.

### Montage sur neutres

On réalise l'opération dans un lit d'air fluidisé GLATT GPCG-3 équipé d'un insert Würster (« bottom spray »).

1500 grammes de théophylline sont mis en suspension dans 4500g d'isopropanol dans lequel on a préalablement solubilisé 262.5 grammes de PVP K29 c'est-à-dire de polyvinylpyrrolidone ou povidone, K29, la valeur de K exprimant le poids moléculaire moyen des povidones calulée d'après la viscosité relative dans l'eau et définie dans la Pharmacopée Européenne (section 2.2.1.2 et 2.3.2.1), utilisé comme agent liant [soit 17%(w/w) par rapport à la masse de théophylline].

On introduit, dans le lit d'air fluidisé, 1500 grammes de neutres constitués d'amidon et de saccharose, d'une taille de l'ordre de 250-350 µm.

On pulvérise sur les neutres la susdite suspension alcoolique contenant la théophylline.

### Enrobage à l'éthylcellulose N7

On réalise l'opération dans un lit d'air fluidisé GLATT GPCG-3 équipé d'un insert Würster (« bottom spray »).

On enrobe 600 grammes de granules obtenus à l'étape précédente par pulvérisation d'une solution d'éthylcellulose N7 dans l'alcool isopropylique telle que préparée à l'exemple 1, contenant en outre du talc micronisé, correspondant à 10%(w/w), par rapport à la masse sèche de polymère.

On applique une quantité de polymère correspondant à 5%(w/w), calculée en gain de poids par rapport à la masse de granules à enrober.

La répartition granulométrique des particules enrobées obtenues et désignées par E2 est indiquée dans le Tableau 6 :

**Tableau 6**

| Ouverture de maille | E2 |
|---|---|
| >0.500mm | 6.6% |
| 0.425mm-0.500mm | 46.2% |
| 0.355mm-0.425mm | 38.2% |
| 0.250mm-0.355mm | 9.0% |

### Surenrobage protecteur

On réalise l'opération dans un lit d'air fluidisé GLATT GPCG-3 équipé d'un insert Würster (« bottom spray »).

On enrobe les granules enrobés E2, obtenus à l'étape précédente, par pulvérisation d'une solution aqueuse de PEG 4000 contenant en outre du talc micronisé correspondant à 10%(w/w), par rapport à la masse sèche de PEG.

On applique l'enrobage protecteur en une quantité correspondant à 20%(w/w), calculée en gain de poids par rapport à la masse de granules E2.

La répartition granulométrique des particules enrobées E3 obtenues par application de l'enrobage protecteur sur les granules E2, est indiquée dans le Tableau 7 :

**Tableau 7**

| Ouverture de maille | E3 |
|---|---|
| >0.500mm. | 15.8% |
| 0.425mm-0.500mm | 41.2% |
| 0.355mm-0.425mm | 38.4% |
| 0.250mm-0.355mm | 4.6% |

On établit un profil de dissolution comparatif entre les granules enrobés E2 et E3 dans les conditions suivantes :
- appareil : USP type II
- vitesse des pales : 100 tours par minute
- volume : 900 ml
- température: 37.0°C ±0.5°C
- détection: Spectrophotométrie UV directe à 272 nm
- milieu de dissolution : HCl 0.01 N (pH = 2)

Les résultats enregistrés sont réunis dans le Tableau 8 :

**Tableau 8**

| | Théophylline libérée (%(w/w) | |
|---|---|---|
| Temps (heure) | **E2** | **E3** |
| 1 | 12 | 10 |
| 2 | 21 | 16 |
| 4 | 36 | 34 |
| 6 | 48 | 49 |
| 8 | 57 | 60 |
| 10 | 63 | 68 |

### Conclusion

De l'examen des résultats réunis dans le Tableau 8, il résulte que l'application de l'enrobage protecteur sur les granules enrobés E2, n'en modifie pas de façon significative le profil de dissolution.

### Exemple 4 :

Comprimés orodispersibles comprenant de la théophylline comme principe actif.

### Compression

On utilise une machine à comprimer alternative "Manesty F3 press" équipée de poinçons POLO de 12mm de diamètre.

Les granules enrobés obtenus selon l'exemple 3 sont mélangés avec des excipients de compression, selon les deux compositions indiquées au Tableau 9, fournissant après compression des comprimés C3 et C4.

**Tableau 9**

| | **C3** | **C4** |
|---|---|---|
| **Constituants** | **% en poids** | **% en poids** |
| Granules enrobés de théophylline E2 ou E3 | 35 | 35 |
| Mannitol 300 | 26.5 | 26.5 |
| Mannitol 60 | 26.5 | 26.5 |
| Crospovidone | 10.0 | 10.0 |
| Arôme | -- | -- |
| Syloid® 244FP | 0.5 | 0.5 |
| Stéarate de magnésium | 1.0 | 1.0 |
| Aspartame | -- | -- |

Les caractéristiques des comprimés multiparticulaires orodispersibles C3 et C4 sont indiquées au Tableau 10 :

**Tableau 10**

| Caractéristiques | C3 | C4 |
|---|---|---|
| Dosage en substance active du comprimé (mg) | 80 | 70 |
| Poids du comprimé (mg) | 588.0 | 622.0 |
| Dureté (kP) | 3.8 | 3.1 |
| Friabilité (%) | 5.6 | 0.5 |
| Temps de désintégration en bouche (secondes) | 29 | 35 |

On établit un profil de dissolution comparatif dans les conditions de l'exemple 3, entre les comprimés orodispersibles multiparticulaires C3 et C4, contenant respectivement les granules enrobés E2 et E3.

Les résultats enregistrés sont réunis dans le Tableau 11 :

**Tableau 11**

| | Théophylline libérée (%(w/w) | |
|---|---|---|
| Temps (heures) | **C3 (E2)** | **C4 (E3)** |
| 1 | 51 (12) | 16 (10) |
| 2 | 67 (21) | 24 (16) |
| 4 | 83 (36) | 43 (34) |
| 6 | 91 (48) | 55 (49) |
| 8 | 95 (57) | 63 (60) |
| 10 | 98 (63) | 69 (68) |

### Conclusion

L'examen des résultats réunis au Tableau 11, permet de déduire que l'enrobage protecteur améliore significativement les propriétés mécaniques du film d'éthylcellulose N7 en termes de résistance à la compression.

### Exemple 5 :

On prépare des comprimés orodispersibles comprenant de la théophylline comme principe actif.

### Compression

On utilise une machine à comprimer alternative "Manesty F3 press" équipée de poinçons POLO de 12mm de diamètre.
Les granules enrobés E3 obtenus selon l'exemple 3 sont mélangés à des excipients de compression selon les indications du Tableau 12, fournissant des comprimés multiparticulaires désignés par C5 :

**Tableau 12**

| | **C5** |
|---|---|
| **Constituants** | **% en poids** |
| Granules enrobés de théophylline E3 | 20 |
| Mannitol 300 | 32.8 |
| Mannitol 60 | 32.8 |
| Crospovidone | 10.0 |
| Arôme | 1.0 |
| Syloid® 244FP | 0.5 |
| Stéarate de magnésium | 1.0 |
| Aspartame | 2.0 |

Les comprimés multiparticulaires orodispersibles C5 ont les caractéristiques résultant du Tableau 13 :

**Tableau 13**

| | **C5** |
|---|---|
| Dosage du comprimé (mg) en substance active | 25mg |
| Poids (mg) | 555.0 |
| Dureté (kP) | 2.5 |
| Friabilité (%) | 0.6 |
| Temps de désintégration en bouche (secondes) | 26 |

On établit un profil de dissolution comparatif dans les conditions de l'exemple 3, entre les granules enrobés E3 et les comprimés multiparticulaires orodispersibles C5 contenant lesdits granules enrobés.

Les résultats enregistrés sont réunis dans le Tableau 14 :

**Tableau 14**

| | Théophylline libérée (%(w/w)) |
|---|---|
| Temps (heures) | **C5 (E3)** |
| 1 | 21 (12) |
| 2 | 30 (21) |
| 4 | 43 (36) |
| 6 | 52 (48) |
| 8 | 60 (57) |
| 10 | 65 (63) |

On établit un profil de dissolution comparatif des comprimés multiparticulaires orodispersibles C5 dans des milieux de dissolution différents, de pH respectivement à 1.2, 4.5 et 6.8 :
Les résultats enregistrés sont réunis dans le Tableau 15 :

**Tableau 15**

| | Théophylline libérée (%(w/w)) | | |
|---|---|---|---|
| Temps (heures) | **pH1,2** | **pH 4.5** | **pH 6.8** |
| 1 | (21) | 21 | 22 |
| 2 | (30) | 31 | 31 |
| 4 | (43) | 44 | 44 |
| 6 | (52) | 53 | 54 |
| 8 | (60) | 60 | 62 |
| 10 | (65) | 66 | 68 |

### Conclusion

De l'examen des résultats réunis au Tableau 15, il est possible de conclure que l'enrobage protecteur améliore significativement les propriétés mécaniques du film d'éthylcellulose N7 en termes de résistance à la compression.

Il est également possible de constater que les profils obtenus sont indépendants du pH du milieu dans lequel est effectuée la dissolution.

### Exemple 6 :

Pour évaluer les différences existant entre les résistances à la compression dans le cas des polymères cellulosiques d'une part, et acryliques d'autre part, on réalise le même essai que dans les exemples 3 et 4, en utilisant comme polymère d'enrobage un polyacrylate connu sous la marque Eudragit® NE30D et commercialisé par RÖHM.

### Enrobage à l'Eudragit® NE30D

On réalise l'opération dans un lit d'air fluidisé GLATT GPCG-3 équipé d'un insert Würster (« bottom spray »).

On enrobe 750 grammes de granules obtenus après montage sur neutres de l'exemple 3, par pulvérisation d'une dispersion aqueuse d'Eudragit®NE30D, diluée à 20%(w/w), contenant en outre du talc micronisé, en quantité correspondant à 10%(w/w), par rapport à la masse sèche de polymère.

On applique une quantité de polymère correspondant à 5%(w/w), calculée en gain de poids par rapport à la masse de granules à enrober.

A la fin de l'enrobage, on réalise une étape supplémentaire de maturation du film à 60°C pendant 2 heures.

On obtient ainsi des particules désignées par E4 et dont la répartition granulométrique est donnée dans le Tableau 16 :

**Tableau 16**

| Ouverture de maille | **E4** |
|---|---|
| >0.500mm | 20.6% |
| 0.425mm-0.500mm | 46.2% |
| 0.355mm-0.425mm | 26.4% |
| 0.250mm-0.355mm | 6.8% |

### Mise en place d'un enrobage protecteur

On réalise l'opération dans un lit d'air fluidisé GLATT GPCG-3 équipé d'un insert Würster (« bottom spray »).

On enrobe les granules enrobés E4 obtenus à l'étape précédente par pulvérisation d'une solution aqueuse de PEG 4000 contenant, en outre, du talc micronisé, en une proportion correspondant à 10%(w/w) par rapport à la masse sèche de PEG.

On applique une quantité d'enrobage protecteur correspondant à 20%(w/w), calculée en gain de poids par rapport à la masse de granule E4.

On obtient ainsi des particules enrobées E5 dont la répartition granulométrique est donnée dans le Tableau 17 :

**Tableau 17**

| Ouverture de maille | **E5** |
|---|---|
| >0.600mm | 4.2% |
| 0.500mm-0.600mm | 23.6% |
| 0.425mm-0.500mm | 43.2% |
| 0.355mm-0.425mm | 24.2% |
| 0.250mm-0.355mm | 4.8% |

On établit un profil de dissolution comparatif entre les granules enrobés E4 et E5 dans les conditions de l'exemple 3.
Les résultats enregistrés sont réunis dans le Tableau 18 :

**Tableau 18**

| | Théophylline libérée (%(w/w) | |
|---|---|---|
| Temps (heure) | **E4** | **E5** |
| 1 | 34 | 40 |
| 2 | 49 | 57 |
| 4 | 66 | 74 |
| 6 | 75 | 84 |
| 8 | 81 | 90 |
| 10 | 86 | 94 |

### Conclusion

De l'examen des données réunies dans le Tableau 18, il apparaît que l'application d'un enrobage protecteur sur les granules E4 n'en modifie pas de façon significative le profil de dissolution.

### Exemple 7:

Comprimés multiparticulaires orodispersibles comprenant de la théophylline comme principe actif

### Compression

On utilise une machine à comprimer alternative "Manesty F3 press" équipée de poinçons POLO de 12mm de diamètre.

Les granules enrobés E4 et E5 obtenus selon l'exemple 6 sont mélangés avec des excipients de compression selon la composition du Tableau 19 et on obtient des comprimés C6 et C7, correspondant respectivement aux granulés enrobés E4 et E5 :

**Tableau 19**

| | **C6** | **C7** |
|---|---|---|
| | % en poids | % en poids |
| Granules enrobés de théophylline | 35 | 35 |
| Mannitol 300 | 26.5 | 26.5 |
| Mannitol 60 | 26.5 | 26.5 |
| Crospovidone | 10.0 | 10.0 |
| Arôme | -- | -- |
| Syloid® 244FP | 0.5 | 0.5 |
| Stéarate de magnésium | 1.0 | 1.0 |
| Aspartame | -- | -- |

Les caractéristiques des comprimés C6 et C7 sont réunies dans le Tableau 20 :

**Tableau 20**

| | **C6** | **C7** |
|---|---|---|
| Dosage du comprimé (mg) en substance active | 100 | 70 |
| Poids (mg) | 680.0 | 583.0 |
| Dureté (kp) | 4.2 | 3.3 |
| Friabilité (%) | 0.8 | 0.5 |
| Temps de désintégration en bouche (secondes) | 14 | 35 |

On établit un profil de dissolution comparatif dans les conditions de l'exemple 3, entre les comprimés orodispersibles multiparticulaires C6 et C7, contenant respectivement les granules enrobés E4 et E5 :
Les résultats enregistrés sont réunis dans le Tableau 21 :

**Tableau 21**

| | Théophylline libérée (%(w/w) | |
|---|---|---|
| Temps (heures) | **C6 (E4)** | **C7 (E5)** |
| 1 | 46 (34) | 48 (40) |
| 2 | 65 (49) | 66 (57) |
| 4 | 82 (66) | 83 (74) |
| 6 | 90 (75) | 92 (84) |
| 8 | 94 (81) | 98 (90) |
| 10 | 97 (86) | 100 (94) |

### Conclusion

De l'examen des données réunies dans le Tableau 21, il résulte que l'enrobage protecteur n'améliore pas les propriétés mécaniques du film formé par l'Eudragit®NE30D qui est déjà souple et déformable. Ceci est attesté par la faible variation observée entre les profils de dissolution des comprimés orodispersibles C6 et C7, contenant respectivement les granules enrobés E4 et E5.

### Exemple 8 :

### Compression

On utilise une machine à comprimer alternative "Manesty F3 press" équipée de poinçons POLO de 12mm de diamètre.

Les granules enrobés E5 obtenus selon l'exemple 6 sont mélangés avec des excipients de compression selon la composition du Tableau 22 où les comprimés mutiparticulaires obtenus sont désignés par C8 :

**Tableau 22**

| | **C8** |
|---|---|
| | % en poids |
| Granules enrobés de théophylline E5 | 20 |
| Mannitol 300 | 32.8 |
| Mannitol 60 | 32.8 |
| Crospovidone | 10.0 |
| Arôme | 1.0 |
| Syloid® 244FP | 0.5 |
| Stéarate de magnésium | 1.0 |
| Aspartame | 2.0 |

Les caractéristiques des comprimés multiparticulaires orodispersibles C8 sont réunies dans le Tableau 23 :

**Tableau 23**

| | **C8** |
|---|---|
| Dosage du comprimé (mg) en substance active | 40 |
| Poids (mg) | 580.0 |
| Dureté (kP) | 2.7 |
| Friabilité (%) | 0.4 |
| Temps de désintégration en bouche (secondes) | 27 |

On établit un profil de dissolution comparatif dans les conditions de l'exemple 3, entre les granules enrobés E5 et les comprimés orodispersibles C8, contenant lesdits granules enrobés E5 :
Les résultats enregistrés sont réunis le Tableau 24 :

**Tableau 24**

| | Théophylline libérée (%(w/w)) |
|---|---|
| Temps (heures) | **C8 (E5)** |
| 1 | 45 (40) |
| 2 | 65 (57) |
| 4 | 85 (74) |
| 6 | 95 (84) |
| 8 | 100 (90) |
| 10 | 100 (94) |

On établit par ailleurs un profil de dissolution comparatif des comprimés multiparticulaires orodispersibles C8 dans trois milieux de dissolution, respectivement à pH de 1.2, 4.5 et 6.8 :
Les résultats figurent dans le tableau 25 :

**Tableau 25**

| | Théophylline libérée (%(w/w)) | | |
|---|---|---|---|
| Temps (heures) | **PH 1.2** | **pH 4.5** | **pH 6.8** |
| 1 | (45) | 45 | 46 |
| 2 | (65) | 64 | 65 |
| 4 | (85) | 84 | 84 |
| 6 | (95) | 94 | 93 |
| 8 | (100) | 100 | 99 |
| 10 | (100) | 100 | 100 |

### Conclusion

A l'examen des résultats réunis dans le Tableau 25, il apparaît que le profil de dissolution n'est pas significativement modifié par la compression et que les profils obtenus sont indépendants du pH du milieu dans lequel est effectuée la dissolution.

## Revendications

1. Comprimé multiparticulaire comprenant :
- un agent de désintégration, et/ou un agent gonflant,
- au moins un agent diluant,
- un lubrifiant, et
- éventuellement, un agent antistatique, un agent perméabilisant, des édulcorants, des arômes et des colorants,
**caractérisé par le fait qu'**il est à base de particules enrobées à libération prolongée, comprenant
- un noyau comprenant un principe actif et au moins un agent liant,
- un film d'enrobage constitué d'au moins un polymère cellulosique, seul ou en mélange avec un plastifiant, et
- un enrobage protecteur à base d'au moins un agent thermoplastique dont la température de fusion est d'environ 25°C à environ 100°C et qui est appliqué sur le film d'enrobage à base d'au moins un polymère cellulosique, ledit agent thermoplastique étant choisi dans le groupe comprenant les huiles partiellement hydrogénées, la cire d'abeille, la cire de carnauba, les cires de paraffine, les cires de silicone, les alcools gras, les acides gras en C12-C18, les glycérides semi-synthétiques solides, les monoester, diesters ou triesters du glycérol, les polyoxyéthylèneglycols, les glycérides polyoxyéthylénés glycosylés, et leurs mélanges.

2. Comprimé multiparticulaire selon la revendication_1, **caractérisé en ce que** l'enrobage protecteur des particules enrobées à libération prolongée est choisi parmi les excipients pour lesquels la balance hydrophile/lipophile (HLB) est supérieure à 10.

3. Comprimé multiparticulaire selon l'une des revendications 1 et 2, **caractérisé en ce qu'**il comprend un liant, un agent perméabilisant, des édulcorants, des arômes et des colorants et que l'enrobage protecteur des particules enrobées comprend un agent antistatique et un lubrifiant.

4. Comprimé multiparticulaire selon l'une des revendications 1 et 3, **caractérisé en ce que** la proportion de mélange d'excipients par rapport aux particules enrobées est de 0,4 à 10, de préférence de 1 à 5 parties en poids.

5. Comprimé multiparticulaire selon l'une des revendications 1 et 4, **caractérisé en ce qu'**il se désagrège dans la bouche au contact de la salive en moins de 60 secondes, de préférence en moins de 40 secondes, en formant une suspension aisée à avaler.

6. Comprimé multiparticulaire selon la revendication 5, **caractérisé en ce qu'**il comprend au moins un agent de désintégration, un agent diluant, un agent lubrifiant et éventuellement un agent gonflant, un agent perméabilisant, des édulcorants et des arômes.

7. Comprimé multiparticulaire selon l'une des revendications 5 et 6, **caractérisé en ce que** l'agent diluant est choisi parmi les agents solubles à propriétés liantes, constitués par un polyol de moins de 13 atomes de carbone se présentant soit sous forme de produit directement compressible dont le diamètre moyen des particules est de 100 µm à 500 µm, soit sous la forme d'une poudre dont le diamètre moyen des particules est inférieur à 100 µm, ce polyol étant de préférence choisi dans le groupe comprenant le mannitol, le xylitol, le sorbitol et le maltitol, utilisés sous la forme du produit directement compressible alors que, dans le cas où il y aurait au moins deux agents solubles diluants à propriétés liantes, l'un est présent sous la forme directement compressible et l'autre sous la forme d'une poudre, le polyol pouvant être le même, les proportions de polyol directement compressible et de polyol poudre étant de 99/1 à 20/80, de préférence de 80/20 à 20/80.

8. Comprimé multiparticulaire selon l'une des revendications 1 à 7 **caractérisé en ce que** les particules enrobées comprennent au moins un principe actif choisi parmi ceux du groupe comprenant les sédatifs gastrointestinaux, les antiacides, les antalgiques, les antiinflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les antidiarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinausééux, les hématopoïétiques, les uricosuriques, les extraits de végétaux, les agents de contraste.

9. Comprimé multiparticulaire selon l'une des revendications 1 à 8 **caractérisé en ce que** l'agent liant entrant dans la constitution des particules enrobées est choisi dans le groupe comprenant les polymères cellulosiques, les polymères acryliques, les povidones, les copovidones, les polyvinylalcools, l'acide alginique, l'alginate de sodium, l'amidon, l'amidon prégélatinisé, les sucroses et leurs dérivés, la gomme guar, les polyéthylèneglycols et leurs mélanges.

10. Comprimé multiparticulaire selon l'une des revendications 1 à 9 **caractérisé en ce que** :
- le noyau des particules enrobées comprend un agent diluant et un agent antistatique,
- le polymère cellulosique comporté par les particules enrobées est l'éthylcellulose,
- le film d'enrobage des particules enrobées comprend un agent formant des pores, un agent plastifiant, un agent tensioactif, un agent antistatique et un lubrifiant, et
- une couche polymérique est appliquée entre le noyau et le film polymérique fonctionnel comportés par les particules enrobées.

11. Comprimé multiparticulaire selon l'une des revendications 1 à 10, **caractérisé en ce que** le diamètre des particules enrobées est de 150 µm à 700 µm, étant entendu qu'au moins 50%, de préférence au moins 70% des particules ont un diamètre de 150 µm à 500 µm, et que moins de 15% des particules ont un diamètre inférieur à 150 µm.

12. Procédé de préparation des particules enrobées comportées par le comprimé multiparticulaire selon l'une des revendications 1 à 11, **caractérisé en ce qu'**il comprend les étapes suivantes :
- préparation, par granulation humide ou montage sur neutres, d'un noyau comprenant le principe actif,
- enrobage des noyaux ainsi obtenus par pulvérisation de la composition d'enrobage constituée d'au moins un polymère cellulosique,
- enrobage des particules enrobées ainsi obtenues par pulvérisation d'une composition d'enrobage protecteur constituée d'un excipient de type thermoplastique, dissous dans un solvant aqueux exempt de solvant organique,
- séchage.

## Claims

1. A multiparticulate tablet comprising
- a disintegrant and/or a swelling agent,
- at least one diluent,
- a lubricant, and
- optionally, an antistatic agent, a permeabilizer, sweeteners, flavorings and colorants,
**characterized in that** it is based on sustained-release coated particles comprising
- a core comprising an active principle and at least one binder, and
- a coating film consisting of at least one cellulosic polymer, alone or as a mixture with a plasticizer, and
- a protective coating based on at least one thermoplastic agent with a melting point of from about 25°C to about 100°C and which is applied to the coating film based on at least one cellulosic polymer, said thermoplastic agent being chosen from the group comprising partially hydrogenated oils, beeswax, carnauba wax, paraffin waxes, silicone waxes, fatty alcohols, C₁₂-C₁₈ fatty acids, solid semisynthetic glycerides, glyceryl monoesters, diesters or triesters, polyoxyethylene glycols and polyoxyethylenated glycosyl glycerides, and mixtures thereof.

2. The multiparticulate tablet as claimed in claim 1,
**characterized in that** the protective coating of the sustained-release coated particle is chosen from excipients chose hydrophilic/lipophilic balance (HLB) is greater than 10.

3. The multiparticulate tablet as claimed in either of claims 1 and 2, **characterized in that** it comprises a binder, a permeabilizer, sweeteners, flavorings and colorants and **in that** the protective coating of the coated particles comprises an antistatic agent and a lubricant.

4. The multiparticulate tablet as claimed in either of claims 1 and 3, **characterized in that** the proportion of excipients mixture relative to the coated particles is from 0.4 to 10 and preferably from 1 to 5 parts by weight.

5. The multiparticulate tablet as claimed in either of claims 1 and 4, **characterized in that** it disintegrates in the mouth on contact with the saliva in less than 60 seconds and preferably in less than 40 seconds, forming a suspension that is easy to swallow.

6. The multiparticulate tablet as claimed in claim 5, **characterized in that** it comprises at least one disintegrant, a diluent, a lubricant and optionally a swelling agent, a permeabilizer, sweeteners and flavorings.

7. The multiparticulate tablet as claimed in either of claims 5 and 6, **characterized in that** the diluent is chosen from soluble agents with binding properties, consisting of a polyol of less than 13 carbon atoms and being either in the form of a directly compressible product with a mean particle diameter of from 100 to 500 µm, or in the form of a powder with a mean particle diameter of less than 100 µm, this polyol preferably being chosen from the group comprising mannitol, xylitol, sorbitol and maltitol, used in the form of a directly compressible product, whereas, in the case where there are at least two soluble diluents with binding propertied, one is present in the directly compressible form and the other in the form of a powder, the polyol possibly being the same, the proportions of directly compressible polyol and of polyol powder being from 99/1 to 20/80 and preferably from 80/20 to 20/80.

8. The multiparticulate tablet as claimed in one of claims 1 to 7, **characterized in that** the coated particles comprise at least one active principle chosen from those of the group comprising gastrointestinal sedatives, antacids, analgesics, antiinflammatories, coronary vasodilators, peripheral and cerebral vasodilators, antiinfectives, antibiotics, antiviral agents, antiparasitic agents, anticancer agents, anxiolytics, neuroleptics, central nervous system stimulants, antidepressants, antihistamines, antidiarrheal agents, laxatives, dietary supplements, immunodepressants, hypocholesterolemiants, hormones, enzymes, antispasmodics, antianginal agents, medicinal products that affect the heart rate, medicinal products used in the treatment of arterial hypertension, antimigraine agents, medicinal products that affect blood clotting, antiepileptics, muscle relaxants, medicinal products used in the treatment of diabetes, medicinal products used in the treatment of thyroid dysfunctions, diuretics, anorexigenic agents, antiasthmatics, expectorants, antitussive agents, mucoregulators, decongestants, hypnotics, antinausea agents, hematopoietic agents, uricosuric agents, plant extracts and contrast agents.

9. The multiparticulate tablet as claimed in one of claims 1 to 8, **characterized in that** the binder included in the constitution of the coated particles is chosen from the group comprising cellulosic polymers, acrylic polymer, povidones, copovidones, polyvinyl alcohol, alginic acid, sodium alginate, starch, pregelatinized starch, sugars and derivative thereof, guar gum and polyethylene glycols, and mixtures thereof.

10. The multiparticulate tablet as claimed in one of claims 1 to 9, **characterized in that**:
- the core of the coated particles comprises a diluent and an antistatic agent,
- the cellulosic polymer borne by the coated particles is ethylcellulose,
- the coating film of the coated particles comprises a pore-forming agent, a plasticizer, a surfactant, an antistatic agent and a lubricant, and
- a polymer layer is applied between the core and the functional polymer film borne by the coated particles.

11. The multiparticulate tablet as claimed in one of claims 1 to 10, **characterized in that** the diameter of the coated particles is from 150 to 700 µm, given that the diameter of at least 50% and preferably of at least 70% of the particles is between 150 and 500 µm, and that the diameter of less than 15% of the particles is less than 150 µm.

12. A process for preparing coated particles borne by the multiparticulate tablet as claimed in one of claims 1 to 11, **characterized in that** it comprises the following steps:
- preparation by wet granulation or mounting on neutral supports, of a core comprising the active principle,
- coating of the cores thus obtained by splaying the coating composition consisting of at least one cellulosic polymer,
- coating of the coated particles thus obtained by spraying with a protective coating composition consisting of an excipient of thermoplastic type, dissolved in an aqueous solvent devoid of organic solvent,
- drying.

## Patentansprüche

1. Multipartikuläre Tablette, umfassend:
- ein Sprengmittel und/oder ein Quellmittel,
- wenigstens ein Verdünnungsmittel
- ein Gleitmittel, und
- gegebenenfalls ein Antistatikum, einen Permeabilisator, Süßstoffe, Aromastoffe und Farbstoffe,
**dadurch gekennzeichnet, dass** sie auf umhüllten Partikeln mit verlängerter Freisetzung basiert, umfassend
- einen Kern, der einen Wirkstoff und wenigstens ein Bindemittel umfasst,
- einen Überzugsfilm, der aus wenigstens einem Cellulosepolymer besteht, allein oder als Mischung mit einem Weichmacher, und
- einen Schutzüberzug, der auf wenigstens einem thermoplastischen Mittel basiert, dessen Schmelztemperatur ungefähr 25°C bis ungefähr 100°C ist und der auf dem Überzugsfilm, auf Basis wenigstens eines Cellulosepolymers aufgebracht ist, wobei das thermoplastische Mittel ausgewählt ist aus der Gruppe umfassend teilweise gesättigte Öle, Bienenwachs, Carnaubawachs, Paraffinwachse, Silikonwachse, Fettalkohole, C12-C18-Fettsäuren, halbsynthetische feste Glyceride, Glycerinmonoester, -diester oder -triester, Polyoxyethylenglykole, polyoxyethylenierte glykosylierte Glyceride, und Mischungen davon.

2. Multipartikuläre Tablette nach Anspruch 1, **dadurch gekennzeichnet, dass** der Schutzüberzug der umhüllten Partikel mit verlängerter Freisetzung ausgewählt ist aus Hilfsstoffen, deren Hydrophil-Lipophil-Gleichgewichtswert (HLB-Wert) größer als 10 ist.

3. Multipartikuläre Tablette nach einem der Ansprüche 1 und 2, **dadurch gekennzeichnet, dass** sie ein Bindemittel, einen Permeabilisator, Süßstoffe, Aromastoffe und Farbstoffe umfasst und dass der Schutzüberzug der umhüllten Partikel ein Antistatikum und ein Gleitmittel umfasst.

4. Multipartikuläre Tablette nach einem der Ansprüche 1 bis 3, **dadurch gekennzeichnet, dass** der Anteil der Hilfsstoffmischung bezogen auf den umhüllten Partikeln 0,4 bis 10, bevorzugt 1 bis 5 Gewichtsanteile beträgt.

5. Multipartikuläre Tablette nach einem der Ansprüche 1 bis 4, **dadurch gekennzeichnet, dass** sie im Mund bei Kontakt mit Speichel in weniger als 60 Sekunden, bevorzugt in weniger als 40 Sekunden, zerfällt, wobei eine leicht zu schluckende Suspension entsteht.

6. Multipartikuläre Tablette nach Anspruch 5, **dadurch gekennzeichnet, dass** sie wenigstens ein Sprengmittel, ein Verdünnungsmittel, ein Gleitmittel und gegebenenfalls ein Quellmittel, einen Permeabilisator, Süßstoffe und Aromastoffe umfasst.

7. Multipartikuläre Tablette nach einem der Ansprüche 5 und 6, **dadurch gekennzeichnet, dass** das Verdünnungsmittel ausgewählt ist aus löslichen Mitteln mit Bindemitteleigenschaften, die aus einem Polyol mit weniger als 13 Kohlenstoffatomen bestehen, das entweder in Form eines Produktes vorliegt, das direkt verpresst werden kann und dessen mittlerer Partikeldurchmesser 100 bis 500 µm ist, oder in Form eines Pulvers, dessen mittlerer Partikeldurchmesser kleiner als 100 µm ist, wobei dieses Polyol bevorzugt ausgewählt ist aus der Gruppe umfassend Mannitol, Xylitol, Sorbitol und Maltitol, die in Form des direkt verpressbaren Produkts verwendet werden, wobei, wenn wenigstens zwei lösliche Verdünnungsmittel mit Bindemitteleigenschaften vorliegen, eines in der direkt verpressbaren Form vorliegt und das andere in Form von Pulver vorliegt, wobei das Polyol das gleiche sein kann und das Verhältnis von direkt verpressbarem Polyol zu Polyolpulver 99/1 bis 20/80, bevorzugt 80/20 bis 20/80, ist.

8. Multipartikuläre Tablette nach einem der Ansprüche 1 bis 7, **dadurch gekennzeichnet, dass** die umhüllten Partikel wenigstens einen Wirkstoff umfassen, ausgewählt aus denen der Gruppe umfassend gastrointenstinale Sedativa, Antazida, Analgetika, Entzündungshemmer, koronare Vasodilatoren, periphere und cerebrale Vasodilatoren, Antiinfektionsmittel, Antibiotika, antivirale Mittel, Antiparasitika, Antikrebsmittel, Anxiolytika, neuroleptische Mittel, Stimulantien des zentralen Nervensystems, Antidepressiva, Antihistaminika, Antidiarrhoika, Laxativa, Nahrungsergänzungsmittel, Immunodepressiva, cholesterinsenkende Mittel, Hormone, Enzyme, Antispastika, Antianginika, Medikamente, die den Herzrhythmus beeinflussen, Medikamente zur Behandlung von arterieller Hypertonie, Antimigränemittel, Medikamente, die die Blutgerinnung beeinflussen, Antiepileptika, Muskelrelaxantien, Medikamente zur Behandlung von Diabetes, Medikamente zur Behandlung von Schilddrüsenstörungen, Diuretika, Anorektika, Antiasthmatika, schleimlösende Mittel, Antitussiva, schleimregulierende Mittel, Dekongestiva, Hypnotika, Mittel gegen Übelkeit, Hämatopoetika, Urikosurika, Pflanzenextrakte und Kontrastmittel.

9. Multipartikuläre Tablette nach einem der Ansprüche 1 bis 8, **dadurch gekennzeichnet, dass** das im Aufbau der umhüllten Tabletten enthaltene Bindemittel ausgewählt ist aus der Gruppe umfassend Cellulosepolymere, Acrylpolymere, Povidone, Copovidone, Poylvinylalkohole, Alginsäure, Natriumalginat, Stärke, vorgelatinierte Stärke, Zucker und deren Derivate, Guaran und Polyethylenglykole, und deren Mischungen.

10. Multipartikuläre Tablette nach einem der Ansprüche 1 bis 9, **dadurch gekennzeichnet, dass**
- der Kern der umhüllten Partikel ein Verdünnungsmittel und ein Antistatikum umfasst,
- das Cellulosepolymer der umhüllten Partikel Ethylcellulose ist,
- der Überzugsfilm der umhüllten Partikel ein porenbildendes Mittel, einen Weichmacher, ein Tensid, ein Antistatikum und ein Gleitmittel umfasst, und
- eine Polymerschicht zwischen dem Kern und dem funktionellen Polymerfilm der umhüllten Partikel aufgebracht ist.

11. Multipartikuläre Tablette nach einem der Ansprüche 1 bis 10, **dadurch gekennzeichnet, dass** der Durchmesser der umhüllten Partikel 150 µm bis 700 µm ist, wobei wenigstens 50%, bevorzugt wenigstens 70%, der Partikel einen Durchmesser von 150 µm bis 500 µm haben, und wenigstens 15% der Partikel einen Durchmesser kleiner als 150 µm haben.

12. Verfahren zur Herstellung von umhüllten Partikeln der multipartikulären Tablette nach einem der Ansprüche 1 bis 11, **dadurch gekennzeichnet, dass** es die folgenden Schritte umfasst:
- Herstellen eines Kerns, der den Wirkstoff umfasst, durch Feuchtgranulierung oder Aufbau auf einem neutralen Träger
- Überziehen der auf diese Weise erhaltenen Kerne durch Aufsprühen der Überzugszusammensetzung, die aus wenigstens einem Cellulosepolymer besteht,
- Überziehen der auf diese Weise erhaltenen Kerne durch Aufsprühen einer schützenden Überzugszusammensetzung, die aus einer Thermoplastmasse besteht, die in einem wässrigen Lösungsmittel, das frei von organischen Lösungsmitteln ist, gelöst ist.
- Trocknen.
